# EUROPEAN PATENT SPECIFICATION

(11) **EP 0 495 547 B1**
(45) Date of publication and mention of the grant of the patent: **24.04.1996**
(21) Application number: 92200058.3
(22) Date of filing: 10.01.1992
(51) Int. Cl.: C07C 51/14, C07C 67/38, C07C 45/50

(54) **Carbonylation of olefins**
Carbonylierung von Olefinen
Carbonylation d'oléfines

(30) Priority: 15.01.1991 GB 9100801; 30.08.1991 GB 9118631; 12.03.1991 GB 9105211
(43) Date of publication of application: 22.07.1992
(73) Proprietor: SHELL INTERNATIONALE RESEARCH MAATSCHAPPIJ B.V., NL-2596 HR Den Haag (NL)
(72) Inventor: Drent, Eit, NL-1031 CM Amsterdam (NL); van Kragtwijk, Eric, NL-1031 CM Amsterdam (NL); Pello, Dennis Humphrey Louis, NL-1031 CM Amsterdam (NL)

(56) References cited:
- EP-A- 0 227 160
- EP-A- 0 406 848
- US-A- 3 168 553

## Description

This invention relates to a process for the monocarbonylation of optionally substituted olefinically unsaturated compounds by reaction with carbon monoxide and a coreactant in the presence of a catalyst system comprising cationic palladium and a bidentate diphosphine. The invention further relates to certain catalytic systems suitable for this monocarbonylation reaction.

A review of various carbonylation reactions is provided by J. Falbe, "New Syntheses with Carbon Monoxide", Springer Verlag, Berlin Heidelberg New York, 1980.

One commercially relevant carbonylation reaction using a hydride source as coreactant, is the hydroformylation of olefins, which are reacted with carbon monoxide and hydrogen in the presence of transition metal catalysts to form aldehydes having one more carbon atom than the precursor olefin. Depending on catalyst, reaction conditions and substrates, the hydroformylation may proceed with varying selectivities to the several possible isomeric aldehydes and varying yields when side reactions occur to a smaller or larger extent. The formation of ketones involving two equivalents of olefin in the reaction with carbon monoxide and hydrogen, is a known side reaction, but generally it does not become the main reaction for olefins higher than ethylene. While cobalt and rhodium catalysts are best documented, recent attention also was focused on palladium catalysts.

Another commercially relevant carbonylation reaction, which uses a nucleophile with a mobile H-atom as coreactant, is the so-called Reppe reaction involving the addition of carbon monoxide to olefinic bonds. Depending on the nature of the coreactant the Reppe reaction presents routes for the manufacture of saturated acids, anhydrides, esters, amides, thioesters, etc. from a wide variety of feedstocks. Historically, this reaction involved transition metal carbonyls, such as Ni(CO)₄, or their precursors. More recent work shows other transition metals including palladium, to be catalytically active.

A particular group of palladium catalysts is disclosed by GB-A-2058074 and comprises cationic palladium complexes having a molecular structure: 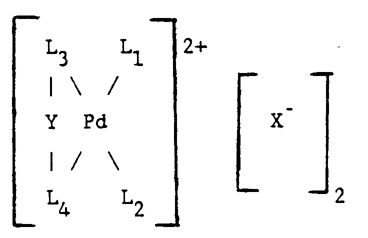
wherein L₁ and L₂ are weakly coordinated solvento-ligands and where L₃-Y-L₄ is a bidentate ligand containing groups L₃ and L₄ each of which has a Group V or Group VI donor atom attached to the palladium atom and further carrying two or one optionally substituted hydrocarbyl groups, and Y is a bridging group having 1-3 atoms in the bridge. The preferred bidentate ligand is 1,2-bis(diphenylphosphino) ethane and the catalyst is proposed for use in alcoholysis and hydrogenation reactions.

The use of a palladium catalyst system comprising a source of palladium cations, a source of bidentate diphosphine, and a source of anions derived from a strong acid, in the preparation of aldehydes is disclosed in EP-A-220767. According to its Examples these catalysts comprising tetraaryl alkylenediphosphine ligand, effect a good reaction rate and high selectivity to aldehydes, in particular linear aldehydes. A further carbonylation reaction using the tetraaryl bidentate diphosphine catalyst system is disclosed in GB-A-2185740 describing the preparation of a mixture of ketones by reaction of olefins with carbon monoxide and hydrogen. The reaction proceeds at a good rate, but a rather complicated mixture of ketones is obtained. Still a further carbonylation reaction using this known catalyst system is disclosed in US-A-4849542 describing the preparation of oxo-alkanedioic acids or diesters by reaction of carboxy or ester substituted olefins with carbon monoxide and hydrogen. The desired diester ketone is produced with high selectivity, but any indication of applicability of the process to olefins carrying other functional groups is absent.

A Reppe reaction for the carbonylation of an olefin with carbon monoxide in the presence of water, an alcohol or a carboxylic acid as coreactant is disclosed in EP-A-55875, wherein a catalyst system comprising palladium and a triorganophosphine containing at least one aliphatic carbon atom bound to phosphorus is used. Whereas for monophosphines PR₃ it is recommended that all groups R represent the same, preferably unsubstituted alkyl group for economic reasons, for any diphosphines, in which the linking alkylene bridge provides the at least one aliphatic carbon atom bound to phosphorus, it is hinted at aromatically substituted diphosphines such as bis(diphenylphosphino)methane only.

It is known that Reppe reactions, wherein an alkenoic acid derivative is carbonylated by reaction with carbon monoxide and a nucleophilic compound having a mobile hydrogen atom, are catalysed by a Group VIII metal such as cobalt. For example, EP-A-143911 discloses the preparation of succinic diesters starting from an acrylic ester and using a cobalt carbonyl complex as catalyst. This known process suffers from the disadvantage of requiring rather severe reaction conditions, such as a carbon monoxide pressure of 120-130 bar. A further process using a cobalt catalyst is described in JP-A-83/72539. In EP-A-274795 a process is described for the carbonylation of olefinically unsaturated compounds with a palladium catalyst. The exemplified catalyst systems comprise a source of palladium, a triarylphosphine, a strong acid and a stabiliser, such as phosphine oxides and sulphides, or tertiary amides. According to its specification the olefinic substrate may be substituted with a broad class of functional groups, including acid, ester, acid amide and acid nitrile groups, but no experimental data on the achievable conversions and selectivities are given.

Where an interesting activity of the above bidentate diphosphine catalyst system, as exemplified by the tetraaryl alkylenediphosphines, in monocarbonylation reactions is apparent, there remains a need for further improved catalyst systems exhibiting still higher reaction rates and selectivities, and applicability to a broad class of olefinic substrates.

It is remarked, that a catalytic system comprising a nonionic compound of palladium and an aliphatic bidentate diphosphine is described by Y. Ben-David et al., in J.Am.Chem.Soc., 1989, 111, 8742-4, and in EP-A-406,848, but only for use in the carbonylation of aryl chlorides.

It was now found that a selected group of catalyst systems characterized by the choice of bidentate diphosphine ligand, offers unexpected advantages over the above systems containing tetraaryl alkylenediphosphines.

Accordingly, the invention provides a process for the monocarbonylation of optionally substituted olefinically unsaturated compounds by reaction with carbon monoxide and a coreactant in the presence of a catalytic system comprising:-
a) a source of palladium cations,
b) a bidentate diphosphine, and
c) a source of anions
   characterized in that the diphosphine is selected from the group of diphosphines of formula I,

   R¹R²P-X-PR³R⁴ (I)

   wherein R¹, R², R³ and R⁴ independently represent an optionally substituted aliphatic group, or R¹ together with R² and/or R³ together with R⁴ represent an optionally substituted bivalent aliphatic group, with the proviso of at least one of said monovalent or bivalent aliphatic groups representing an optionally branched or cyclic alkyl or alkylene having at least one alpha hydrogen atom; and X represents a bivalent bridging group containing 1 to 10 atoms in the bridge, with the exception of 1,4-bis(dicyclohexylphosphino)butane.

It is remarked that the last proviso relates to a change anticipation in EP-A-227,160 discussed on page 2, and the first proviso relates to copending patent application EP-A-495 848.

The unexpected advantages of the present invention will be demonstrated by the Examples and Comparative Examples hereinafter. In direct comparison with the tetraaryl diphosphines, the present catalyst systems comprising aliphatic diphosphines provide substantially higher reaction rates, yields and/or selectivities to desired products in various monocarbonylation reactions. Moreover, where appropriate, a surprising high regioselectivity to desired isomers was observed. Also, the present process appears to be applicable to a broad class of optionally substituted olefinically unsaturated compounds.

Apart from any specific catalytic mechanism, the gross reaction of the present monocarbonylation process involves the respective insertions of a molecule of carbon monoxide and at least one molecule of olefinically unsaturated compound into a hydrogen bond of the coreactant. Representing the coreactant by NuH, wherein Nu represents the remnant nucleophilic moiety of the coreactant after removal of a hydrogen atom, it is seen from the gross reaction: 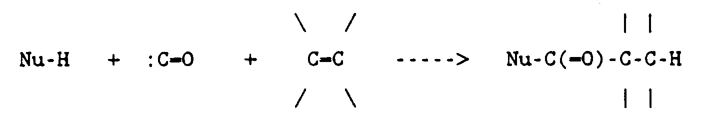
that the nature of the coreactant largely determines the type of product formed. The coreactant can be a nucleophilic compound having a mobile hydrogen atom, such as an alcohol, an acid, an amine or water. For an alcohol ROH, the RO moiety is represented by Nu and accordingly the product is an ester, 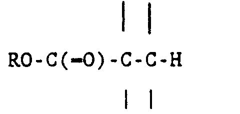
Similarly, the use of an acid RCOOH (Nu = RCOO) will introduce an anhydride group in the product of the monocarbonylation reaction; the use of ammonia (Nu = NH₂) or an amine RNH₂ (Nu = RNH) or R₂NH (Nu = R₂N) will introduce an amide group; the use of a thiol RSH (Nu = RS) will introduce a thioester group; and the use of water (Nu = OH) will introduce a carboxy group. Realizing that the hydrogen-hydrogen bond of molecular hydrogen is polarisable, it will be readily appreciated that the coreactant can also be molecular hydrogen, or more generally a hydride source, in which case the monocarbonylation reaction is frequently referred to as hydroformylation for mostly historical reasons.

An accepted mechanism for transition metal (cobalt or rhodium) catalysed monocarbonylation reactions of the hydroformylation type involves a catalytically active species comprising a transition metal hydride bond, into which successively the C=C moiety of an olefin molecule, a carbon monoxide carbon atom, and optionally the C=C moiety of a further olefin molecule or a second C=C moiety of the same polyolefin molecule are inserted. The catalytic cycle is terminated by reaction of the intermediate with a hydride source, whereby a metal hydride bond is reformed with expulsion of the monocarbonylation product. The palladium catalysed reaction is similarly believed to involve palladium hydride bonds. The hydride source conveniently is molecular hydrogen, in which case the process may be fed with a readily available carbon monoxide and hydrogen mixture such as synthesis gas. However, the hydride source may also be a source being able to generate molecular hydrogen under the process conditions, such as water in view of the equilibrium CO + H₂O = CO₂ + H₂. In some cases, the hydride source may be constituted by the reactant itself, if the intermediate palladium-alkyl is prone to beta-hydrogen abstraction by the palladium atom, as demonstrated in Example 4b and 5d hereinafter.

Though less extensively studied, the other carbonylation reactions are also believed to involve insertion of an olefinic group and carbon monoxide into a palladium hydride bond, and subsequent termination of the catalytic cycle by the nucleophilic moiety Nu. Another reaction mechanism involves initial coordination to palladium of the nucleophilic moiety Nu, subsequent insertion of carbon monoxide and an olefinic group, and termination of the catalytic cycle by protons or the next molecule of the coreactant.

Suitable alcohols, acids, or amines used as coreactant, may be aliphatic, cycloaliphatic or aromatic, and may be substituted or unsubstituted. Representative examples include methanol, ethanol, propanol, butanol, phenol, acetic acid, stearylalcohol, benzylalcohol, cyclohexanol, cresol, propionic acid, butyric acid, pivalic acid, aniline, and p-anisidine. Preference is given to nucleophilic compounds having 1 to 12 carbon atoms.

If the starting olefinically unsaturated compound comprises a substituent acid function Y', it will be appreciated that the NuCO function formed may be the same as the function Y' of the precursor, such as in dimethylsuccinate obtained from the carbonylation of methyl acrylate in the presence of methanol, or may be of the same type, as in ethylmethylsuccinate obtained either from the carbonylation of methylacrylate in the presence of ethanol, or from the carbonylation of ethylacrylate in the presence of methanol.

NuCO and Y' may also be different types of acid functions, such as in monomethylsuccinate obtained from the carbonylation of methylacrylate in the presence of water. The skilled man will appreciate that the alternative route of carbonylation of acrylic acid in the presence of methanol may be liable to competitive reactions involving the acrylic acid itself functioning as nucleophilic compound NuH, with consequent decrease of selectivity. Preferably, the reactants are selected such, that the nucleophilic compound is more reactive than the acid function of the alkenoic acid derivative in order to suppress the occurrence of such competitive reactions.

It is known that, under circumstances, a plurality of carbon monoxide and olefin molecules may be inserted within a single catalytic cycle, whereby macromolecular products are formed. Higher olefins and functionally substituted olefins in general show less tendency to undergo polycarbonylation reactions. However, using lower olefins, such as ethene and to a lesser extent propene, polycarbonylation reactions may well occur. As used in the present specification and claims the term "monocarbonylation" expressly is intended to exclude such processes involving plural carbonyl insertions or "polycarbonylations". Accordingly, the present process is to be carried out substantially under monocarbonylating conditions. As will be apparent from the above, any undesired polycarbonylation reaction can be readily suppressed by favouring the reactions involving termination of a catalytic cycle, more particularly by favouring the metal hydride bond formation. Accordingly, the present process is preferably carried out under conditions of high hydride activity. Means of increasing the hydride activity are known to the skilled artisan, and include the increase of partial hydrogen pressure, the increase of temperature and the use of active hydride sources.

In any event, the Examples in this specification provide a clear guidance to the skilled man in applying reaction conditions for directing the carbonylation to the formation of the specific desired product.

Without wishing to be bound by any theory, it is believed that the active catalyst species in the catalyst system is a cationic complex of palladium with the diphosphine of formula I. Accordingly, the catalyst system employed in the process according to the invention must comprise a source of palladium cation, a source of the diphosphine, and a source of an anion, it being understood that the combined sources of catalyst components may develop catalytic activity in the initial stage of the process only, or in some cases after an induction period. The remaining coordination sites at the central palladium atom are believed to be exchangeably occupied by molecules of carbon monoxide, olefin substrate, solvent, anion and product intermediates.

The source of palladium cation is preferably a palladium salt. Examples of salts include salts of nitric acid; sulphuric acid; sulphonic acids, for example chlorosulphonic acid, methanesulphonic acid, trifluoromethane sulphonic acid, t-butylsulphonic acid, p-toluenesulphonic acid, or a sulphonated ion exchange resin; and a carboxylic acid, for example an alkanoic acid such as acetic acid or trifluoro acetic acid. Since halide ions can be corrosive, the source of palladium cation is preferably not a halide.

It will be appreciated that when the source of palladium cation is a palladium salt of a suitable acid, it will also be a source of an anion. It will also be appreciated that for the source of palladium cation, the palladium may be present in a complex, for example with the diphosphine. The source of palladium cation may therefore also be a source of a diphosphine of formula I hereinbefore.

The source of palladium cation may also be the metallic element or a palladium compound other than a salt. For example, it may be an oxide or a zero valent complex with a ligand such as a phosphine or carbon monoxide. When the source of palladium cation is not a salt, it should be used with a protonic acid. It will be appreciated that the protonic acid can also be the source of an anion derived from a strong acid, except hydrohalogenic acids.

The quantity of the source of palladium cation is not critical. Preferably it is sufficient to provide in the range of from 10⁻⁷ to 10⁻¹ gram atoms of Pd per mole of olefinically unsaturated compound, more preferably from 10⁻⁶ to 10⁻².

The source of a diphosphine of formula I is conveniently the diphosphine itself, or an acid addition salt of the diphosphine. It may also be a complex of the diphosphine with palladium.

In formula I of the diphosphine to be used according to the present invention,

R¹R²P-X-PR³R⁴ (I),

each of R¹, R², R³ and R⁴ independently represents an optionally substituted aliphatic group, suitably having from 1 to 20 carbon atoms, or one or both pairs of R¹ and R², and R³ and R⁴ independently represent an optionally substituted bivalent aliphatic group. Preferred aliphatic groups are unsubstituted optionally branched or cyclic alkyl or alkylene groups having from 1 to 10 carbon atoms, more preferably from 1 to 6 carbon atoms. Examples of suitable alkyl groups are methyl, ethyl, n-propyl, iso-propyl, n-butyl, sec-butyl, iso-butyl, tert-butyl, cyclohexyl and n-hexyl. Preferred alkyl or alkylene groups have one or two alpha-hydrogen atoms, in particular one alpha-hydrogen atom as in secondary alkyl and alkylene groups. Most preferred alkyl group are ethyl, i-propyl, n-propyl, i-butyl and n-butyl. Examples of suitable alkylene groups include hexamethylene and cyclooctylene.

When the alkyl group is said to be optionally substituted, it may be substituted by one or more substituents which do not annihilate the catalytic activity of the system. Suitable substituents include halogen atoms, alkoxy groups, haloalkyl groups, haloalkoxy groups, acyl groups, acyloxy groups, amino groups, hydroxyl groups, nitrile groups, acylamino groups, and aryl groups.

As the diphosphine of formula I is believed to be coordinated to palladium in bidentate mode, it should have the two phosphine P atoms at intramolecular distance and configuration allowing coordination to a single metal atom. Accordingly, any bridge connecting the two phosphorus atoms should be free of any substituents causing hindrance to metal coordination. The bridging group represented by X is preferably a hydrocarbon, an ether or a thioether residue. For example, the bridging group may be an alkylene chain which is optionally interrupted by one or more oxygen and/or sulphur atoms, as in:
-CH₂-; -CH₂CH₂-; -CH₂CH₂CH₂-; CH₂OCH₂-; CH₂SCH₂-; -CH₂CH₂CH₂CH₂-; -CH₂CH₂OCH₂CH₂-; -CH₂CH₂SCH₂CH₂-; -CH₂CH₂OCH₂CH₂OCH₂CH₂-; 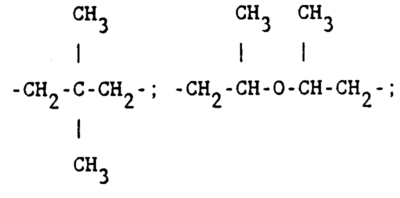
or a silane residue:

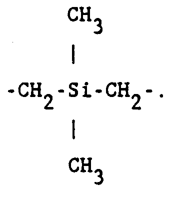

The bridging group preferably contains from 2 to 8 atoms in the bridge, more preferably from 3 to 5 atoms. For example, when the bridging group is a propane or neopentane residue, the bridge contains 3 atoms. Most preferably the bridging group is a trimethylene, tetramethylene or 3-oxapentamethylene group.

Examples of diphosphines of formula I which may be used in the monocarbonylation according to the invention are:
1,2-bis(di-n-butylphosphino)ethane, 1,3-bis(dimethylphosphino)propane, 1,3-bis(diethylphosphino)propane, 1,3-bis(di-i-propylphosphino)propane, 1,3-bis(di-n-propylphosphino)propane, 1,3-bis(di-i-butylphosphino)propane, 1,3-bis(di-n-butylphosphino)propane, 1,3-bis(di-s-butylphosphino)propane, 1,3-bis(di-n-hexylphosphino)propane, 1,2-bis(dicyclohexylphosphino)ethane, 1,3-bis(n-butylmethylphosphino)propane, 1,3-bis(n-butylethylphosphino)propane, 1,3-bis(1,5-cyclooctylenephosphino)propane and its isomeric mixture containing 1,4-cyclooctylene groups, 1,4-bis(di-i-propylphosphino)butane, 1,5-bis(dimethylphosphino)-3-oxapentane, 1,8-bis(di-n-butylphosphino)-3,6-dioxaoctane, and 1,4-bis(di-n-butylphosphino)-2,2,3,3-tetramethylbutane.

The ratio of the number of moles of the diphosphine of formula I per gram atom of palladium is preferably in the range of from 0.50 to 10, more preferably from 0.9 to 5, especially from 1 to 3.

The source of an anion, used in the process of the invention is preferably a protonic acid. However, as mentioned hereinabove, it may also be a salt of palladium. It may also be a salt of another metal, for example of vanadium, chromium, nickel, copper or silver.

Preferably the anion is a non- or weakly-coordinating anion: that is to say an anion which does not or only weakly coordinates with the palladium cation. It is preferably derived from a strong acid having a pKa < 3.5 (measured at 18 °C in aqueous solution). Since halide anions, in particular chloride anion, tend to coordinate fairly strongly to palladium, the anion preferably is derived from strong acids except hydrohalogenic acids. The anion may also be derived from a weak acid, for example a carboxylic acid, in which case it is preferred that the carboxylic acid is sterically hindered, in that it comprises bulky substituents in the proximity of the carboxy group. Thus, any too strong coordination tendency is mitigated. Anions derived from weak carboxylic acids are particularly suitable for catalyst systems used in the monocarbonylation of unsubstituted alpha-olefins in the presence of an alcohol to form an ester or the presence of an amine to form an amide.

For example, the anion may be derived from nitric acid; sulphuric acid; a sulphonic acid such as fluorosulphonic acid, chlorosulphonic acid, methanesulphonic acid, 2-hydroxypropanesulphonic acid, t-butylsulphonic acid, p-toluenesulphonic acid, benzenesulphonic acid, trifluoromethanesulphonic acid, or a sulphonated ion exchange resin; a perhalic acid such as perchloric acid; a perfluorated carboxylic acid such as trifluoroacetic acid; orthophosphoric acid; a phosphonic acid such as benzenephosphonic acid; or an acid derived by the interaction of a Lewis acid, such as BF₃, PF₅, AsF₅, SbF₅, TaF₅ or NbF₅, with a Broensted acid, such as HF (e.g. fluorosilicic acid, HBF₄, HPF₆, HSbF₆). Carboxylic acids, from which suitable anions may be derived, include 2,4,6-trimethylbenzoic acid, 2,6-dichlorobenzoic acid, 9-anthroic acid, pivalic acid, 1,2,3-benzenetricarboxylic acid and its 1,3-diester which may be formed in situ, 2-ethoxy-1-naphthalene carboxylic acid, and 2,6-dimethoxybenzoic acid.

Typical non-coordinating anions, by which is meant that little or no covalent interaction occurs between palladium and the anion, include PF₆⁻, SbF₆⁻, BF₄⁻, and ClO₄⁻, and anions derived from an acids having a pKa below 2 (measured at 18 °C in aqueous solution), such as sulphuric acid, sulphonic acids, e.g. an optionally substituted hydrocarbylsulphonic acid such as benzenesulphonic acid, p-toluenesulphonic acid, naphthalenesulphonic acid, an optionally substituted alkylsulphonic acid such as methanesulphonic acid, tert-butylsulphonic acid, 2-hydroxypropanesulphonic acid or trifluoromethanesulphonic acid, chlorosulphonic acid or fluorosulphonic acid, phosphonic acids and carboxylic acids such as trichloroacetic acid or trifluoroacetic acid. The source of anions may also be an acidic ion exchange resin, for example a sulphonated ion exchange resin.

It will be appreciated that when using a palladium salt of a weak acid, such as acetic acid, the addition of a strong acid such as a sulphonic acid will generate a salt of palladium with the stronger acid, and the weak acid.

The ratio of moles of anions per gram atom of palladium is not critical. Preferably it is in the range of from 0.5 to 100, more preferably in the range of from 1 to 10.

The diphosphines of formula I as such are known compounds, and can be prepared by general methods described in the literature, for example Houben-Weyl, Vol. XII/I, p.21.

If an alkenoic acid derivative is carbonylated using as coreactant a nucleophilic compound having a mobile hydrogen atom, it is preferred that the catalyst system further comprises a promoter, suitably an organic oxidant promoter, such as quinones and nitro compounds. Whilst not wishing to be bound by theory, it is believed that the promoter activates the catalytic palladium centre by annihilating any hydrides or hydrogen generated through a shift reaction from traces of water present in the system. Also drying agents, such as trimethyl orthoformate, may be used as promoter. Suitable quinones comprise the ortho- or para-diketo benzene moiety, which may be substituted or be part of a condensed ring system. Examples of suitable quinone promoters include benzoquinones, such as 1,2-benzoquinone, 1,4-benzoquinone, 2-chloro-1,4-benzoquinone, 2,6-dimethyl-1,4-benzoquinone and tetrachloro-p-benzoquinone, naphthoquinones, such as 1,2-nahphthaquinone and 1,4-naphthaquinone, anthraquinones, such as 9,10-anthraquinone, and phenanthroquinones such as 9,10-phenanthroquinone. Mixtures of quinones may be present. The ratio of moles of quinone promoter to palladium is not critical. Preferably it is in the range of from 1 to 1000. more preferably in the range of from 5 to 10.

The catalyst system used in the process according to the invention may be homogeneous or heterogeneous. Preferably it is homogeneous, or an immobilised homogeneous catalyst system.

The catalyst system according to the invention is preferably constituted in a liquid phase. The liquid phase may conveniently be formed by one or more of the reactants with which the catalyst system is to be used. Alternatively, it may be formed by a solvent. It may also be formed by one of the components of the catalyst system.

The olefinically unsaturated compound is preferably a substituted or unsubstituted alkene or cycloalkene, preferably having in the range of from 2 to 30, in particular 3 to 20 and, more particularly, 3 to 10 carbon atoms per molecule, and preferably 1 to 3 carbon-carbon double bonds per molecule. The alkene or cycloalkene may be substituted, for instance with one or more halogen atoms, or cyano, ester, carboxy, amino, amido, nitrile, alkoxy, aryl, or thioalkoxy groups. Examples of olefinically unsaturated compounds are ethene, propene, 1-butene, 2-butene, isobutene, the isomeric pentenes, hexenes, octenes and dodecenes, 1,5-cyclooctadiene, cyclododecene, methyl acrylate, methyl methacrylate, ethyl acrylate, acrylic acid, acrylonitrile, acrylamide, N,N-dialkylacrylamide, vinyl chloride, ethyl vinyl ketone, allyl chloride, methyl allyl ether, styrene, and acrylaldehyde.

Preferably the optionally substituted olefinically unsaturated compound contains at least one terminal olefinic bond, and is an optionally substituted alpha-olefin. Preferred optionally substituted olefinically unsaturated compounds may be represented by the formula II: 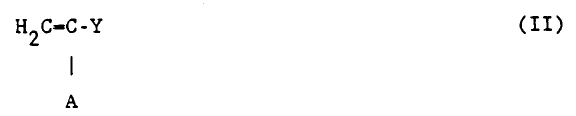
wherein A represents a hydrogen atom or hydrocarbyl, preferably methyl, group, and Y represents hydrogen or an electron-withdrawing or electron-releasing substituent, such as selected from the group of carboxy, nitrile, formyl, amino, halo and substituents of formula -Z-R with Z representing a single valency bond or a functionality -CO-, -COO-, -OOC-, -NH-, -CONH-, -NHCO-, -O-, or -S-; and R representing an optionally substituted alkyl, alkenyl, cycloalkyl, cycloalkenyl, aryl or heterocyclic group. The alkyl group is preferably a C₁₋₁₀ alkyl group, more preferably a C₁₋₆ alkyl group, such as methyl, ethyl, n-propyl, iso-propyl, n-butyl, iso-butyl, tert-butyl and n-hexyl. The cycloalkyl group is preferably a C₃₋₆ alkyl group, for example cyclopentyl or cyclohexyl. The alkenyl and cycloalkenyl groups may have the same number of carbon atoms and a carbon-carbon double bound in any position. The aryl group is preferably a phenyl or a naphthyl group. The heterocyclic group has preferably from 3 to 12 ring atoms including from 1 to 3 hetero atoms such as oxygen, nitrogen and sulphur.

Particularly suitable substrates for the present process when using a nucleophile having a mobile H atom, include alkenoic acid derivatives such as alkenoic acids, alkenoic acid anhydrides, alkenoic acid amides, alkenoic acid nitriles, and alkenoic esters. Preferably, the substrate has the acid functionality directly attached to an olefinic carbon atom, and thus is a 2-alkenoic acid derivative. The alkenyl moiety of the alkenoic acid may be substituted, but is preferably unsubstituted, such as in vinyl, 1-propenyl, 1-butenyl, 1-pentenyl and 1-hexenyl, and preferably has from 2 to 12 carbon atoms. Representative examples of suitable substrates include acrylic acid, methacrylic acid, 2-butenoic acid, 2-pentenoic acid, acrylonitrile, methacrylonitrile, acrylamide, and methacrylamide. Further examples are N-substituted acrylamides and methacrylamides, acrylates, methacrylates, and other esters of the afore-mentioned alkenoic acids. The N-substituents of the amide groups and the O-substituents of the ester groups may be aliphatic, cycloaliphatic or aromatic, and may be substituted or unsubstituted, and preferably have from 1 to 10 carbon atoms. Examples are methyl acrylate, ethyl acrylate, phenyl acrylate, i-propyl acrylate, n-butyl acrylate, and the corresponding methacrylates, and N,N-dimethylacrylamide. Preferably, the substrate alkenoic ester derivative is an alkenoic acid, more preferably an acrylic ester.

Further particularly suitable substrates include unsubstituted alpha-olefins which can be carbonylated in the presence of a nucleophile having a mobile H atom to produce esters or other carbonyl compounds.

When an optionally substituted olefinically unsaturated compound is subjected to monocarbonylation in the process according to the present invention when using a hydride source as coreactant, one or two equivalents may react with one mole of hydrogen and carbon monoxide each. An aldehyde is obtained as the product of the reaction of one equivalent of olefins. A ketone is obtained as the of the reaction of two equivalents of olefin. When producing ketones, two different olefinically unsaturated compounds may be reacted, whereby mixed ketones are obtained.

It will be appreciated that the ketone and aldehyde formations are competitive reactions, and the reaction product might be expected to contain mixtures of both. Surprisingly, in the present process the selectivities to either aldehydes or ketones are high. Additionally, the present invention provides the unexpected flexibility of allowing for direction of the reaction to either ketone or aldehyde formation by proper choice of the anion component of the catalyst system, as is shown for 1-octene in the Examples hereinafter. As a rule, non-coordinating anions, such as trifluoromethylsulphonate, p-tolylsulphonate, tetrafluoroborate, perchlorate, and hexafluorophospate tend to favour ketone formation, whereas weakly coordinating anions, such as trifluoroacetate, and benzenephosphonate tend to favour aldehyde formation.

Our copending EP-A-529,698 discloses the use of a catalyst system comprising a Group VIII metal - preferably palladium - compound, a bidentate diphosphine and, preferably, a source of anions derived from a strong acid in the hydrogenation of an aldehyde to the corresponding alcohol.

It will be appreciated that some catalyst systems are active in both the present process producing aldehydes and the hydrogenation process disclosed in said copending patent application. Accordingly, the aldehydes produced in the present process may in situ be converted into the corresponding alcohols. Under reaction conditions of fast monocarbonylation and slow hydrogenation, the aldehyde may be produced at high concentration in the reaction mixture, from which it could be isolated, if desired. By adapting the reaction conditions to fast hydrogenation, for example by raising the temperature or increasing the hydrogen partial pressure, the intermediate aldehyde is further reacted to the alcohol in the same liquid reaction phase.

By appropriate choice of reaction conditions of fast hydrogenation the alcohol may directly be obtained from an olefinically unsaturated compound as the aldehyde initially formed is immediately consumed in the faster subsequent hydrogenation reaction to form the alcohol.

Alternatively, the course of the reaction may be directed to either the aldehyde product through monocarbonylation or the alcohol product through hydrogenation by varying the coordinative strength of the anion as is seen in the Examples. As a rule, the hydrogenation requires a lower coordinative strength of the anion component of the catalytic system, or a lower pKa value for the associated acid, than is required for the monocarbonylation reaction to proceed. By appropriate choice of the anion component, therefore, the aldehyde product may be prepared with excellent selectivity.

This versatility in obtaining various products at high selectivity and reaction rate using the present catalytic system constitutes a very surprising and advantageous feature of the present invention.

Again, by following the above directories and guided by the Examples hereinafter, the skilled man will readily be able to select the appropriate catalyst components and reaction conditions for obtaining a desired functionalised product starting from an available olefinically unsaturated compound.

When using a hydride source as coreactant, the process according to the invention can be carried out suitably using a molar rate of carbon monoxide to hydrogen of 1:1 which is the stoichiometric ratio to produce aldehydes or ketones. Excess carbon monoxide or hydrogen over the stoichiometric amount may be present, for instance in a molar ratio between 12:1 and 1:12. The gaseous feed of carbon monoxide and hydrogen may be diluted with an inert gas such as nitrogen.

It is not necessary to use a separate solvent in the process according to the invention. The starting olefin and the ester, amide, ketone or aldehyde product can often form a suitable liquid phase. In some cases, however, it may be desirable to use a separate solvent. Any inert solvent can be used for that purpose. Said solvent may, for example, comprise sulphoxides and sulphones, for example dimethylsulphoxide, diisopropylsulphone or tetrahydrothiophene-2,2-dioxide (also referred to as sulfolane), 2-methylsulfolane, 3-methylsulfolane, 2-methyl-4-butylsulfolane; aromatic hydrocarbons such as benzene, toluene, and xylenes; esters such as methyl acetate and butyrolactone; ketones such as acetone or methyl isobutyl ketone; alcohols such as methanol and ethanol, ethers such as tetrahydrofurane (also referred to as THF), anisole, 2,5,8-trioxanonane (also referred to as diglyme), diphenyl ether and diisopropylether; and amides such as dimethylacetamide and N-methylpyrrolidone. Alcohols may function as coreactant to form esters, but may also function as inert solvent in hydroformylation reactions to form aldehydes or ketones, if the hydride activity is sufficiently high.

The process according to the invention is conveniently effected at a temperature in the range of from 10 °C to 200 °C, in particular from 50 °C to 150 °C.

The process according to the invention is preferably effected at a total pressure of from 1 to 80 bar. Pressures higher than 100 bar may be used, but are generally economically unattractive on account of special apparatus requirements. More preferred pressures are in the range of from 5 to 70 bar. Preferred pressures in the range of from 1 to 15 bar are particularly suitable when using a stripping reactor, for example in a process for the carbonylation of alkenes in the presence of an alkanol to form alkyl carboxylates according to EP-A-411721.

According to a further aspect the present invention relates to a use of a catalyst system as specified hereinabove, in the monocarbonylation of optionally substituted olefinically unsaturated compounds.

Some of the catalyst systems used in the process of the invention are new. Accordingly, the invention also relates to a catalyst system comprising
a) a source of palladium cations,
b) a bidentate diphosphine, and
c) a source of anions characterized in that the diphosphine is selected from the group of diphosphines of formula I,

   R¹R²P-X-PR³R⁴ (I)

   wherein R¹, R², R³ and R⁴ independently represent an optionally substituted aliphatic group, or R¹ together with R² and/or R³ together with R⁴ represent an optionally substituted bivalent aliphatic group, with the proviso of at least one of said monovalent or bivalent aliphatic groups representing an optionally branched or cyclic alkyl or alkylene having at least one alpha hydrogen atom; and X represents a bivalent bridging group containing 1 to 10 atoms in the bridge, with the exception of 1,4-bis(dicyclohexylphosphino)butane.

Preferably, each of R¹, R², R³ and R⁴ represents a secondary alkyl group, or each of R¹ and R² together and R³ and R⁴ together represents a cycloalkylene group, such as in 1,3-bis(di-isopropylphosphino)propane or 1,3-bis(1,5-cyclooctylenephosphino)propane.

The process according to the invention may be carried out continuously or batchwise.

The monocarbonylation products prepared by the present process find application as chemical solvent or as precursor for various chemicals.

The invention will now be illustrated by the following Examples.

### Example 1

A 250 mi magnetically-stirred autoclave was charged with 20 ml 1-octene, 50 ml diglyme (2,5,8-trioxanonane), 0.25 mmol of palladium acetate, 0.3 mmol of 1,3-bis(di-n-butylphosphino)propane and 2mmol trifluoroacetic acid. After being flushed, the autoclave was pressurized with carbon monoxide and hydrogen up to a partial pressure of 30 bar of each. The autoclave was sealed, heated to a temperature of 90 °C, and maintained at that temperature for 5 hours, whereupon a sample of the contents of the autoclave was analysed by gas liquid chromatography. From the results of the analysis, the selectivity to nonanals was found to be close to 100% (based upon converted 1-octene), and the conversion rate was calculated to be 120 mol/gram atom Pd/hour. The linearity of the nonanals (percent of n-nonanal on the total of nonanals formed) amounted to 75%.

### Comparative Example A

Example 1 was exactly repeated except for adding 0.3 mmol 1,3-bis(diphenylphosphino)propane instead of the butylated diphosphine. A selectivity of close to 100%, a reaction rate of 50 mol/gram atom Pd/hour, and a linearity of 72% were found.

### Example 2

The procedure of Example 1 was repeated using various olefinically unsaturated substrates, diphosphine ligands, anion sources, and CO/H₂ ratios at the temperatures as indicated in Table 1. In each experiment 0.25 mmol palladium acetate and 50 ml diglyme solvent (40 ml in examples 2b and 2c) were used at a total CO and H₂ pressure of 60 bars during a reaction time of 5 hours. The observed selectivity to aldehyde product, conversion rate and product linearity are mentioned in the Table.

**TABLE 1**

| Ex. No. | Substrate | | Phosphine | | Anion source | | Ratio CO/H₂ | Temp. °C | Sel. % | Conv. rate | Lin. % |
|---|---|---|---|---|---|---|---|---|---|---|---|
| | type | ml | type | mmol | type | mmol | | | | | |
| 2a | 1-C₈⁼ | 20 | 4-Me | 0.30 | CF₃COOH | 2.0 | 1:1 | 90 | 100 | 120 | 72 |
| 2b | 1-C₈⁼ | 20 | 4-iPr | 0.60 | CF₃COOH | 1.0 | 1:1 | 125 | 98 | 150 | 84 |
| 2c | 1-C₈⁼ | 20 | 4-iPr | 0.60 | PhPO₃H₂ | 1.0 | 1:1 | 125 | 98 | 50 | 85 |
| 2d | 1-C₈⁼ | 15 | 4-cHx | 0.60 | tBuSO₃H | 1.0 | 1:1 | 70 | 98 | 100 | 64 |
| 2e | styrene | 20 | 4-iPr | 0.60 | CF₃COOH PTSA 1.0 | 1.0 | 1:2 | 80 | 94* | 150 | 88 |
| 2f | C₃⁼ | 30 | 4-sBu | 0.60 | tBuSO₃H | 1.0 | 7:5 | 80 | 95 | 500 | 84 |
| Legends: 4-Me = 1,3-bis(dimethylphosphino)propane 4-iPr = 1,3-bis(diisopropylphosphino)propane 4-cHx = 1,2-bis(dicyclohexylphosphino)ethane 4-sBu = 1,3-bis(di-sec-butylphosphino)propane PTSA = p-toluenesulphonic acid | | | | | | | | | | | |

| | | | | | | | | | | | |
|---|---|---|---|---|---|---|---|---|---|---|---|
| * 6% selectivity to 3- and 2-phenyl-1-propanols | | | | | | | | | | | |

### Example 3

A 250 ml magnetically-stirred autoclave was charged with 20 ml 1-octene, 40 ml methanol, 0.25 mmol palladium acetate, 0.3 mmol 1,3-bis(di-n-butylphosphino)propane, and 1 mmol paratoluenesulphonic acid. After being flushed, the autoclave was pressurized with carbon monoxide and hydrogen to a partial pressure of 30 bar each. Then the autoclave was sealed, heated to a temperature of 125 °C, and maintained at that temperature for 5 hours, whereupon a sample of the contents of the autoclave was analysed by GLC. On the basis of converted olefin, the reaction product comprised 85% of 7-methyl-8-hexadecanone, 10% of 7-methylene-8-hexadecanone and traces of 6-ethyl-7-pentadecanone, 5-propyl-6-tetradecanone and diketones. The conversion rate of 1-octene was 100 mol/gram atom Pd/hour. It is seen that the reaction proceeded with a high selectivity (95%) to the ketone linking the olefin residues at the alpha and beta carbon atoms respectively, Y-CH₂-CH₂-CO-CHY-CH₃, (Y = hexyl).

### Comparative Example B

Example 3 was exactly repeated using, however, 0.25 mmol 1,3-bis(diphenylphosphino)propane instead of 0.25 mmol 1,3-bis(di-n-butylphosphino)propane. At a conversion rate below 15 mol/gram atom Pd/hour, the formed reaction product comprised 10% of 7-methyl-8-hexadecanone, besides higher branched C₁₇-ketones and unidentified oligomeric products.

### Example 4

a. Example 3 was repeated except for adding 20 ml of styrene instead of 20 ml of 1-octene and heating to a temperature of 100 °C. On the basis of converted styrene, the resulting reaction product comprised 90% of 1,5-diphenyl-3-pentanone, 5% of 1,5-diphenyl-1-penten-3-one, and traces of 1,4-diphenyl-3-pentanone. The rate of conversion of styrene was 125 mol/gram atom Pd/hour. It is seen that the reaction proceeded with a high selectivity to the ketones linking the alpha carbon atoms of both olefin residues, Y-CH₂-CH₂-CO-CH₂-CH₂-Y.
b. A 250 ml magnetically-stirred autoclave was charged with 20 ml styrene, 40 ml methanol, 0.25 mmol palladium acetate, 0.3 mmol 1,3-bis(di-n-butylphosphino)propane, 0.5 mmol Ni(CF₃SO₃)₂, and 5 mmol naphthaquinone. After being flushed, the autoclave was pressurized with 20 bar of carbon monoxide. Then the autoclave was sealed, heated to a temperature of 70 °C, and maintained at that temperature for 5 hours, whereupon a sample of the contents of the autoclave was analysed by GLC. Styrene appeared to be converted at a rate of 40 mol/gram atom Pd/hour to 1,5-diphenyl-1-penten-3-one with a selectivity close to 90%.

### Example 5

a. Example 3 was repeated except for adding 30 ml of propene, instead of 1-octene, 0.1 mmol, instead of 0.25 mmol, palladium acetate, 0.25 mmol, instead of 0.3 mmol, of 1,3-bis(di-n-butylphosphino)propane, and 0.3 mmol of trifluoromethylsulphonic acid, instead of paratoluenesulphonic acid. After a reaction time of 2 hours, the product was found to comprise 67% of 2-methyl-3-hexanone, 15% of 2-methyl-1-hexen-3-one, 15% of other C₇-ketones combined, and traces of diketones on the basis of converted propene. The rate of conversion of propene was 500 mol/gram atom Pd/hour. It is seen that the reaction proceeded with a high selectivity to the ketone linking the olefin residues at the alpha and beta carbon atoms respectively, Y-CH₂-CH₂-CO-CHY-CH₃, (Y = methyl).
b. Repeating the experiment in 50 ml methanol solvent using 2 mmol of paratoluenesulphonic acid as anion component of the catalyst system, after 1 hour at a propene conversion rate of 1500 mol/gram atom Pd/hour, the product comprised 70% 2-methyl-3-hexanone, and 10% 2-methyl-1-hexen-3-one, and 5% of its methanol adduct: 1-methoxy-2-methyl-3-hexanone, besides isomeric C₇-ketones, on the basis of converted propene.
   Excellent regioselectivities to alpha,beta (Examples 3 and 5) or alpha,alpha (Example 4) ketones were obtained in Examples 3-5, in particular, when considering the yields of alkanones and alkenones jointly. In the present context, by alpha linking is meant that the carbonyl group is bonded to the terminal alpha carbon atom of the substrate olefin; similarly, beta linking means that the carbonyl group is bonded to the non-terminal beta carbon atom of the substrate olefin. The ratio of alkanones to alkenones may be controlled by increasing the ratio of hydrogen to carbon monoxide to above 1:1 for higher alkanone content, and below 1:1 for a higher proportion of alkenone.
c. A 250 ml magnetically-stirred autoclave was charged with 30 ml propene, 40 ml diglyme, 0.25 mmol palladium acetate, 0.3 mmol 1,3-bis(di-i-propylphosphino)propane, 1 mmol trifluoromethanesulphonic acid, and 5 ml water. After being flushed, the autoclave was pressurized with 30 bar carbon monoxide, sealed, and heated to a temperature of 115 °C. After a reaction time of 1 hour, a sample of the contents of the autoclave was analyzed by gas liquid chromatography. On the basis of converted propene, the reaction product comprised 65% of 2-methyl-3-hexanone (isopropyl propyl ketone) and 26% of 2-methyl-1-hexen-3-one (isopropenyl propyl ketone). The conversion rate of propene was 1000 mol/gram atom Pd/hour. It is seen that water instead of hydrogen can be used as an effective hydride source.
d. The experiment described under 5c. was repeated using, however, a catalyst system comprising 0.1 mmol palladium acetate, 0.25 mmol 1,3-bis(di-n-butylphosphino)propane, and 0.2 paratoluenesulphonic acid; 40 bar instead of 30 bar carbon monoxide pressure; and 0.5 ml instead of 5 ml water. After heating at 125 °C for 5 hours, a sample of the contents of the autoclave was analyzed by GLC. On the basis of converted propene, the reaction product comprised 31% of 2-methyl-3-hexanone and 56% of 2-methyl-1-hexen-3-one. The conversion rate of propene was 250 mol/gram atom Pd/hour. It seen that the combined selectivity (or regio-selectivity) to iso-C₃ n-C₃ ketones remained about the same (91% and 87%, resp.). However, as a result of the lower amount of water as hydride source, the proportion of saturated ketone had decreased in favour of the unsaturated ketone. It is believed that the intermediate palladium alkyl functioned as hydride source by beta hydrogen elimination in the formation of the unsaturated ketone.

### Comparative Example C

Example 5(a) was repeated except for using a catalyst system comprising 0.1 mmol palladium acetate, 0.25 mmol 1,3-bis(diphenylphosphino)propane, and 2 mmol paratoluenesulphonic acid. The propene was converted at a rate of 500 mol/gram atom Pd/hour. The obtained product mixture comprised 40% of propyl isoprop(en)yl ketones, 15% of diisoprop(en)yl ketones, 10% of dipropyl ketones, and 30% of oligomeric ketones.

### Examples 6-13b and Comparative Example D

A series of experiments was carried out by each time charging a 250 ml magnetically-stirred autoclave with methyl acrylate, a solvent, 0.25 mmol of palladium acetate, 0.30 mmol of a diphosphine, and an anion source (in the form of the acid or salt). After being flushed, the autoclave was pressurized with carbon monoxide and hydrogen to a total pressure of 60 bars. Then the autoclave was sealed, heated for a certain period, whereupon a sample of the contents of the autoclave was analyzed by gas liquid chromatography. The reaction product comprised mainly the alpha,alpha-linked CH₃O-CO-CH₂-CH₂-CO-CH₂-CH₂-CO-OCH₃, minor amounts of the alpha,beta-linked CH₃O-CO-CH(CH₃)-CO-CH₂-CH₂-CO-OCH₃, and various amounts of side products, noticeable hydrogenated substrate. Product selectivities as percent of converted methyl acrylate, and rates of conversion of methyl acrylate substrate were calculated. The nature and amount of substrate and solvent, the type of diphosphine, the type and amount of anion, the ratio of CO/H₂ on the basis of partial pressures ratio, the heating temperature, the reaction time, and the observed selectivities and conversion rates are represented in Table 2. It is seen that the conversion rates are much higher when using a catalyst system comprising an alkylated diphosphine instead of an arylated diphosphine in accordance with the prior art teaching.

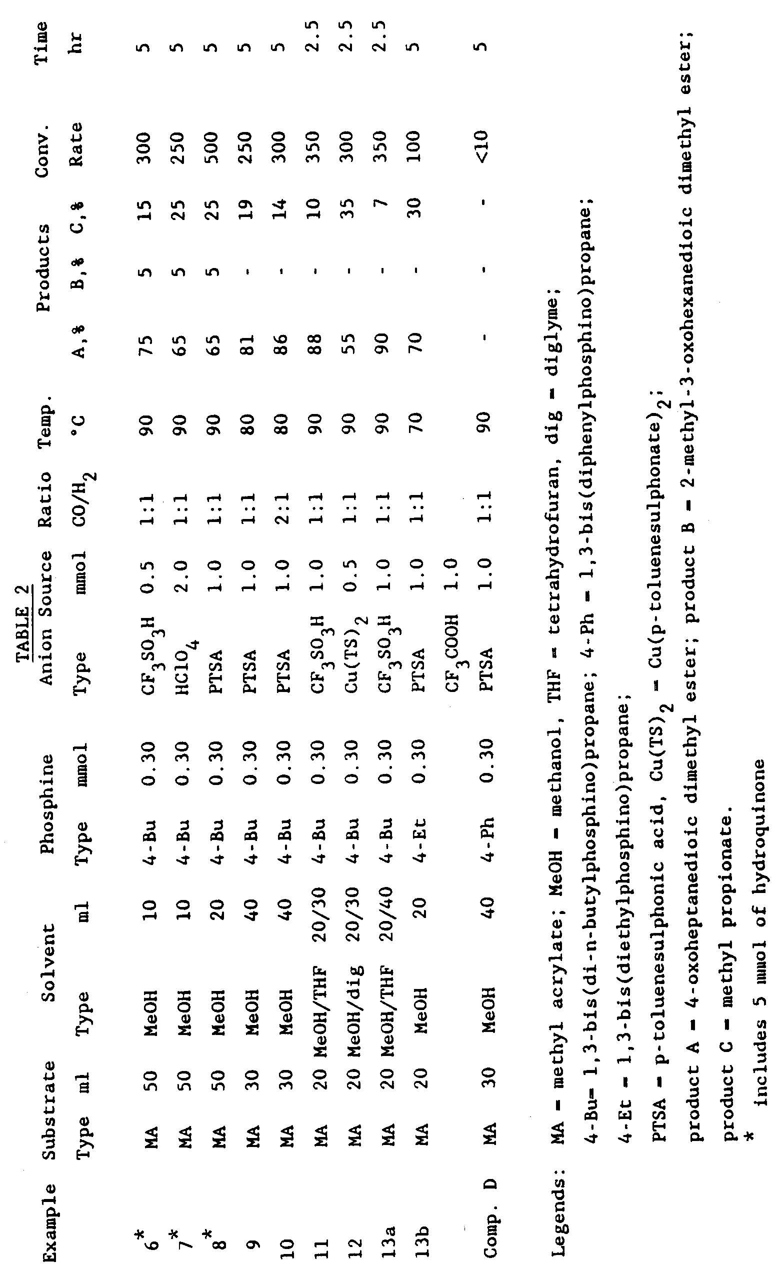

### Example 13c

Using the procedure of Examples 6-13b, 15 ml methyl acrylate in 20 ml methanol and 40 ml diglyme was heated at 90° C for 7 hours under an atmosphere of 30 bar carbon monoxide and 30 bar hydrogen in the presence of a catalyst comprising 0.25 mmol palladium acetate, 0.60 mmol 1,3-bis (diethylphosphino) propane, 1.0 mmol nickel(II) bis(trifluoromethyl sulphonate), and 5 mmol naphthaquinone. A 90% conversion of methyl acrylate with 90% selectivity to 4-oxoheptanedioic dimethyl ester was observed.

### Example 13d

Using the procedure of Examples 6-13b, 10 ml ethyl 3-pentenoate in 40 ml diglyme was heated at 115° C for 5 hours under an atmosphere of 30 bar carbon monoxide and 30 bar hydrogen in the presence of a catalyst comprising 0.25 mmol palladium acetate, 0.60 mmol 1,3-bis (di-s-butylphosphino) propane, and 1.0 mmol t-butylsulphonic acid. A 80% conversion of ethyl 3-pentenoate was observed into ethyl esters of formylpentanoic acids, comprising 84% of the linear ethyl 6-oxohexanoate.

### Example 14

A 250 ml magnetically-stirred autoclave was charged with 20 ml methyl methacrylate, 50 ml THF, 0.25 mmol palladium acetate, 0.30 mmol 1,3-bis (di-n-butylphosphino) propane, 1.0 mmol trifluoromethyl sulphonic acid, and 5 mmol naphthaquinone. After being flushed, the autoclave was pressurized with 30 bar of hydrogen and carbon monoxide each, sealed and heated to 90 °C for a period of 5 hours. Afterwards a sample of the contents of the autoclave was analyzed by gas liquid chromatography. The methyl methacrylate was converted at a rate of 150 mol/gram atom Pd/hour to give 30% yield of 2,6-dimethyl-4-oxoheptanoic dimethyl ester at a conversion of about 40%.

### Comparative Example E

Example 14 was repeated except for using 0.30 mmol of 1,3-bis(diphenylphosphino) propane instead of 0.30 mmol of 1,3-bis(di-n-butylphosphino) propane. After 5 hours reaction time only traces of 2,6-dimethyl-4-oxoheptanoic dimethyl ester were formed.

### Examples 15-19

A series of experiments was carried out by each time charging a 250 ml magnetically-stirred autoclave with a functionally substituted olefin substrate, CH₂=CH-Y, 50 ml tetrahydrofuran, 0.25 mmol of palladium acetate, 0.30 mmol of 1,3-bis(di-n-butyl phosphino)propane, and an anion source in the form of the corresponding acid. After being flushed, the autoclave was pressurized with 30 bar carbon monoxide and 30 bar hydrogen. Then the autoclave was sealed, and heated for a period of 5 hours, whereupon a sample of the contents of the autoclave was analyzed by gas liquid chromatography. The reaction product comprised carbonylation product in the form of the alpha,alpha-linked ketone, Y-CH₂-CH₂-C(=O)-CH₂-CH₂-Y, and hydrogenation product, CH₃-CH₂-Y. Product yields, as percent of the theoretical yield as well as rates of conversion of the substrate, expressed in mol/gram atom Pd/hour, were calculated. Table 3 mentions the nature and amount of substrate, the type and amount of anion, the heating temperature, the conversion rate, and the product yields for each experiment. It is seen that the catalyst system to be used according to the invention, provides for effective carbonylation of a wide scope of substrates to produce di-functionally substituted ketones.

**TABLE 3**

| Example No. | Substrate CH₂=CH-Y | | Temp. °C | Anion Source | | Conv. rate | Carbon. product yield,% | Hydrog. product yield,% |
|---|---|---|---|---|---|---|---|---|
| | Y | ml | | Type | mmol | | | |
| 15¹⁾ | -COOH | 20 | 80 | CF₃SO₃H | 1.0 | 700 | 75²⁾ | 20 |
| 16 | -CONH₂ | 15(g) | 90 | CF₃SO₃H | 1.0 | 100 | 30³⁾ | 30 |
| 17 | -CN | 20 | 90 | CF₃SO₃H | 1.0 | 100 | 20 | 30 |
| 18 | -COCH₂CH₃ | 20 | 90 | CF₃SO₃H | 0.5 | 250 | 40 | 20 |
| 19 | -COH | 20 | 90 | CF₃SO₃H | 0.5 | 100 | 20 | 20 |

| | | | | | | | | |
|---|---|---|---|---|---|---|---|---|
| ¹⁾ solvent: 40 ml THF/10 ml water, 1 hour reaction time | | | | | | | | |
| ²⁾ isolated as di-spiro-lacton through internal condensation | | | | | | | | |
| ³⁾ isolated as di-spiro-lactam through internal condensation | | | | | | | | |

### Example 20

A 250 ml magnetically-stirred autoclave was charged with 25 ml 1-octene, 15 ml methyl acrylate, 30 ml THF, 0.25 mmol palladium acetate, 0.30 mmol 1,3-bis(di-n-butylphosphino)propane and 0.5 mmol trifluoromethylsulphonic acid. After being flushed the autoclave was pressurized with 30 bar carbon monoxide and 30 bar hydrogen. The autoclave was sealed, heated to a temperature of 90 °C, and maintained at that temperature for 5 hours, whereupon a sample of the contents of the autoclave was analyzed by gas liquid chromatography. The reaction product comprised octyl methoxycarbonylethyl ketone formed with a selectivity of 98% based on 1-octene and a selectivity of 60% based on methyl acrylate and 4-oxoheptanedioic dimethylester with a selectivity of 30% based on methyl acrylate. The mixed ketone showed 96% linearity to 4-oxododecanoic methyl ester. The rate of formation of combined ketones was 150 mol/gram atom Pd/hour.

### Example 21

Example 20 was repeated except for substituting 15 ml acrylic acid for 15 ml methyl acrylate, and heating to 70 °C for 1 hour. The reaction product comprised 90%-linear 4-oxododecanoic acid formed with a selectivity of about 100% based on 1-octene and about 50% based on acrylic acid, and 2,7-dioxo-1,6-dioxaspiro[4,4]nonane formed with a selectivity of 20% based on acrylic acid. The rate of formation of combined ketones was 350 mol/gram atom Pd/hour.

### Example 22

Example 20 was repeated except for substituting 30 ml isobutene for 25 ml 1-octene, charging 20 ml instead of 15 ml of methyl acrylate, omitting the THF solvent and heating to a temperature of 80 °C. The reaction product comprised 6-methyl-4-oxoheptanoic methyl ester formed with a selectivity of about 100% based on isobutene and about 50% based on methyl acrylate and 4-oxoheptanedioic dimethylester with a selectivity of 40% based on methyl acrylate. The rate of formation of combined ketones was 100 mol/gram atom Pd/hour.

### Example 23

Example 20 was repeated except for substituting 20 ml vinyl acetate for 25 ml 1-octene, using 50 ml THF solvent and heating to 70 °C instead of 90 °C. The reaction product comprised 7-methyl-3,6,9-trioxo-2,8-dioxadecane and 3,6,10-trioxo-2,9-dioxa-undecane as cross-carbonylation products from vinyl acetate and methyl acrylate, and 4-oxoheptanedioic dimethylester side product from the carbonylation of the methyl acrylate.

### Example 24

Example 20 was repeated except for substituting 15g acryl amide for 15 ml methyl acrylate. The reaction product comprised 4-oxododecanoic acid amide as cross-carbonylation product from 1-octene and acryl amide, and 2,7-dioxo-1,6-diazaspiro [4.4] nonane side product from the carbonylation of the acryl amide.

### Example 25

Example 20 was repeated except for substituting 15 ml acrylonitrile for 15 ml methyl acrylate. The reaction product comprised 4-oxododecanoic acid nitrile as cross-carbonylation product from 1-octene and acrylo nitrile, and 4-oxoheptanedioic acid dinitrile side product from the carbonylation of the acrylo nitrile.

### Example 26

A 250 ml magnetically-stirred autoclave was charged with 20 ml α-octene, 40 ml diglyme (2,5,8-trioxanonane), 0.25 mmol of palladium acetate, 0.6 mmol of 1,3-bis(di-i-propylphosphino)propane and 1 mmol t-butylsulphonic acid. After being flushed, the autoclave was pressurized with carbon monoxide and hydrogen up to a partial pressure of 30 bar of each. The autoclave was sealed, heated to a temperature of 70 °C, and maintained at that temperature for 7 hours, whereupon GLC of a sample of the contents of the autoclave showed that 80% of the α-octene had been converted into nonyl aldehydes, of which 88% were linear and 12% were branched.

After cooling the autoclave was flushed, and was then pressurized with 60 bar of hydrogen and heated at 90 °C for 5 hours. GLC analysis showed a 100% conversion of nonyl aldehydes into the corresponding nonyl alcohols at an initial rate of conversion above 300 mol/gr at Pd/hr. The residual octenes remaining after the hydroformylation step, appeared to be substantially unchanged during the hydrogenation, with only 6% being hydrogenated.

### Example 27

a. A 250 ml magnetically-stirred autoclave was charged with 20 ml α-octene, 40 ml diglyme, 0.25 mmol of palladium acetate, 0.6 mmol of 1,3-bis(di-i-propylphosphino)propane and 1 mmol p-toluenesulphonic acid. After being flushed, the autoclave was pressurized with carbon monoxide and hydrogen up to a partial pressure of 30 bar of each. The autoclave was sealed, heated to a temperature of 90 °C, and maintained at that temperature for 5 hours, whereupon GLC analysis of a sample of the contents of the autoclave showed that 67% of the α-octene had been converted with a selectivity of 94% into nonyl aldehydes and 5% into the corresponding nonyl alcohols.
b. The procedure under a. of this Example was repeated charging the autoclave with 15 ml of α-octene and the same solvent and catalytic system. The autoclave was pressurized with 20 bar of carbon monoxide and 40 bar of hydrogen, and heated at 125 °C for 5 hours. GLC analysis showed that 63% of the α-octene had been converted with a selectivity of 88% into nonyl alcohols and 9% into nonyl aldehydes.

It is seen that using the same catalytic system the aldehyde is formed as the predominant product under a., whereas at higher hydrogen pressure and higher temperature the alcohol is the predominant product under b.. Apparently, in both experiments the aldehyde is formed in a first reaction step and subsequently consumed as a starting material for the second hydrogenation step, under a. at relatively low rate and under b. at relatively high rate under conditions of temperature and hydrogen concentration favourable for hydrogenation.

### Examples 28-39

A 250 ml magnet-driven autoclave of Hastelloy C (Trade Mark) was charged with an alkanol and further liquid and solid components of the reaction mixture as stated for each specific experiment. The catalyst components were dissolved in the respective solvent (usually the alkanol) under nitrogen atmosphere in a glove box and introduced in the nitrogen blanketed autoclave. The autoclave was closed, evacuated and subsequently pressurized with carbon monoxide and any other stated gaseous component. The autoclave was then heated to the indicated temperature and maintained at that temperature, usually for a period between 1 and 5 hours. Finally the contents of the autoclave were analysed by gas liquid chromatography.

In Table 4 below the following data are indicated: the type of olefin (in bar, or alternatively in ml); the catalyst components present in the reaction mixture (in mmoles) besides 0.25 mmol palladium acetate used in all experiments; the type of alkanol (in ml); the pressure of the olefin, carbon monoxide (in bar); the reaction temperature (in °C); the nature of the primary reaction product; the rate (in moles of ester produced per gram atom of palladium per hour); and the selectivity with respect to the desired product (in %).

It is seen that the alkanoate esters of various alkanols including isopropanol and tert-butanol are readily produced using a cationic palladium catalyst comprising a bidentate diphosphine. Diphosphines having aliphatic substituents on phosphorus are preferred over those having aromatic substituents in view of the higher reaction rates. Examples 28-39 are within the specific scope of Claim 20.

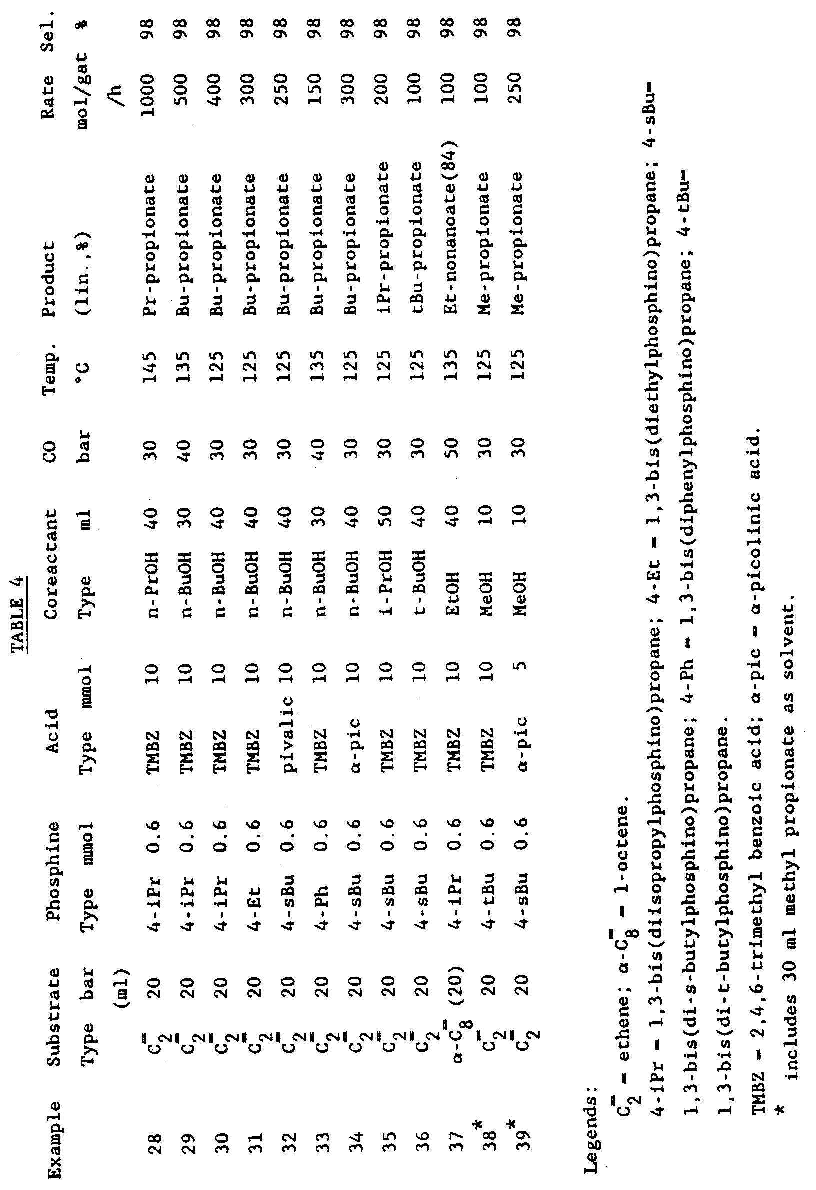

### Example 40

Example 30 was repeated, except for using 5 mmol 1,2,3-benzenetricarboxylic acid instead of TMBZ and a reaction temperature of 135 °C. n-Butyl propionate was formed at a conversion rate of 300 mol/ gat Pd/hour and with a selectivity of 98%.

### Example 41

Example 30 was repeated, except for using 10 mmol 2-ethoxy-1-naphthalene carboxylic acid instead of TMBZ and a reaction temperature of 135 °C. n-Butyl propionate was formed at a conversion rate of 350 mol/ gat Pd/ hour and with a selectivity of 98%.

### Example 42

Example 30 was repeated, except for using 10 mmol 2,6-dimethoxybenzoic acid instead of TMBZ and a reaction temperature of 135 °C. n-Butyl propionate was formed at a conversion rate of 350 mol/ gat Pd/hour and with a selectivity of 98%.

### Example 43

Following the procedure of Examples 28-39, n-butyl propionate was prepared with a selectivity of 98% using 40 ml 1-butanol, 20 bar ethene and 30 bar carbon monoxide as reactants and a catalyst system comprising 0.25 mmol palladium acetate, 0.6 mmol 1,3-bis(di-s-butylphosphino)propane, 10 mmol (20 mmol in experiments a and g) 2,4,6-trimethylbenzoic acid, and the indicated amounts of various sterically hindered bases, if any, at the indicated reaction temperatures. The following conversion rates were observed:

| | | | | |
|---|---|---|---|---|
| 43a | 2,6-di-t-butylpyridine | (10 mmol) | 145 °C | 600 mol/gat/h |
| 43b | 2,6-di-t-butylpyridine | (10 mmol) | 140 °C | 620 mol/gat/h |
| 43c | 2,6-di-t-butylpyridine | (20 mmol) | 120 °C | 275 mol/gat/h |
| 43d | 2,6-di-t-butylpyridine | (10 mmol) | 120 °C | 275 mol/gat/h |
| 43e | - - | - - | 120 °C | 225 mol/gat/h |
| 43f | 3,4-lutidine | (10 mmol) | 120 °C | 140 mol/gat/h |
| 43g | 3,4-lutidine | (10 mmol) | 120 °C | 260 mol/gat/h |

It is seen that the presence of basic compounds did not adversely affect the carbonylation reaction. It should be appreciated that thus the acidity of the reaction mixture by the added anion source can be controlled, what can be advantageous when carbonylating acid-sensitive substrates.

### Example 44

A 250 ml magnetically-stirred autoclave was charged with 10 g phenol, 40 ml diglyme, 0.25 mmol of palladium acetate, 0.6 mmol of 1,3-bis(di-s-butylphosphino)propane and 10 mmol of 2,4,6-trimethylbenzoic acid. After being flushed, the autoclave was pressurized with 20 bar of ethene and 30 bar of carbon monoxide. The autoclave was sealed, heated to a temperature of 130 °C, and maintained at that temperature for 5 hours, whereupon GLC analysis of a sample of the contents of the autoclave showed that 100 % of the phenol had been converted into phenyl propionate at a conversion rate of 150 mol/ gram atom Pd/hour.

### Example 45

A 250 ml magnetically-stirred autoclave was charged with 40 ml of diglyme, 5 g of water (278 mmol), 28.1 g of triethylamine (278 mmol), 0.25 mmol of palladium acetate, 0.6 mmol of 1,3-bis(di-s-butylphosphino)propane and 10 mmol of 2,4,6-trimethylbenzoic acid. After being flushed, the autoclave was pressurized with 20 bar of ethene and 30 bar of carbon monoxide. The autoclave was sealed, heated to a temperature of 150 °C, and maintained at that temperature for 5 hours, whereupon GLC analysis of a sample of the contents of the autoclave showed that propionic acid has been formed at a conversion rate ranging from 150 to 300 mol/ gat Pd/hours.

### Example 46

A 250 ml magnetically-stirred autoclave was charged with 40 ml of diglyme, 10 g of propionic acid (135 mmol), 13.7 g of triethylamine (135 mmol), 0.25 mmol of palladium acetate, 0.6 mmol of 1,3-bis(di-s-butylphosphino)propane and 10 mmol of 2,4,6-trimethylbenzoic acid. After being flushed, the autoclave was pressurized with 20 bar of ethene and 30 bar of carbon monoxide. The autoclave was sealed, heated to a temperature of 150 °C, and maintained at that temperature for 5 hours, whereupon GLC analysis of a sample of the contents of the autoclave showed that 100% of the propionic acid had been converted into propionic anhydride at a conversion rate of 220 mol/ gat Pd/ hour.

### Example 47

A 250 ml magnetically-stirred autoclave was charged with 40 ml of diglyme, 2.5 ml of ethylenediamine, 0.25 mmol of palladium acetate, 0.6 mmol of 1,3-bis(di-s-butylphosphino)propane and 10 mmol of 2,4,6-trimethylbenzoic acid. After being flushed, the autoclave was pressurized with 20 bar of ethene and 30 bar of carbon monoxide. The autoclave was sealed, heated to a temperature of 150 °C, and maintained at that temperature for 10 hours, whereupon GLC analysis of a sample of the contents of the autoclave showed that 100% of the ethylenediamine had been converted into a mixture of amides comprising 10%mol N,N,N',N'-tetrapropionyl ethylenediamine, 40 %mol of N,N,N'-tripropionyl ethylenediamine, and 50 %mol of N,N'-dipropionyl +ethylenediamine. The reaction proceeded at an initially high conversion rate with formation of the diamide, whereas the subsequent steps to tri- and tetraamide using an amide as co-reactant proceeded at a lower rate.

### Examples 48-58

A 250 ml stainless steel autoclave equipped with a magnetic stirrer was each time charged with 20 ml methyl acrylate, 40 ml methanol, 0.25 mmol of palladium acetate and the amounts of further catalyst components as indicated in Table 5. The autoclave was then flushed with carbon monoxide, and then charged with carbon monoxide to a pressure of 40 bar. Subsequently, the autoclave was sealed and heated to a temperature as indicated for a period of 5 or 15 hours, as indicated.

After the indicated reaction time, the contents of the autoclave were analyzed by gas-liquid chromatography (GLC). The observed conversions, expressed as percentages and defined as 100 b/c, in which "b" is the total amount of methyl acrylate that has been converted and "c" is the initial amount of methyl acrylate, and selectivities, expressed as percentages and defined as 100 a/b, in which "a" is the amount of methyl acrylate that has been converted into dimethylsuccinate and "b" again is the total amount of methyl acrylate that has been converted, are represented in Table 5.

**TABLE 5**

| Example No. | Pd(OAc)₂ mmol | phosphine¹⁾^{,} (mmol) | anion source²⁾, (mmol) | quinone³⁾^{,} (mmol) | temperature °C | time hour | conversion % | selectivity % |
|---|---|---|---|---|---|---|---|---|
| 48 | 0.25 | TBPD, (0.3) | NiTFS, (0.5) | NQ, (5) | 100 | 5 | 30 | 99 |
| 49 | 0.25 | TBPD, (0.3) | NiTFS, (0.5) | NQ, (5) | 115 | 5 | 45 | 99 |
| 50 | 0.25 | TBPD, (0.3) | NiTFS, (0.5) | NQ,(10) | 90 | 5 | 15 | 99 |
| 51 | 0.25 | TBPD, (0.3) | NiTFS, (0.5) | NQ,(10) | 90 | 15 | 45 | 99 |
| 52 | 0.25 | TBPD, (0.3) | TFSA, (0.5) | NQ, (5) | 100 | 5 | 20 | 94 |
| 53 | 0.25 | TEPD, (0.3) | NiTFS, (0.5) | NQ, (5) | 90 | 15 | 55 | 98 |
| 54 | 0.25 | PPh₃, (3.0) | PTSA, (2.0) | - | 100 | 5 | 5 | 67 |
| 55 | 0.25 | PPh₃, (3.0) | PTSA, (2.0) | - | 100 | 15 | 7 | 67 |
| 56 | 0.25 | PPh₃, (3.0) | TFSA, (2.0) | NQ, (5) | 100 | 5 | 3 | 55 |
| 57 | 0.25 | TBPD, (0.3) | TFSA, (0.5) | - | 100 | 5 | < 1 | n.d.⁴⁾ |
| 58 | 0.25 | TBPD, (0.3) | NiTFS, (0.5) | - | 90 | 5 | 1 | n.d. |

| | | | | | | | | |
|---|---|---|---|---|---|---|---|---|
| Notes: 1) TBPD = 1,3-bis(di-n-butylphosphino)propane; TEPD = 1,3-bis(diethylphosphino)propane; PPh₃ = triphenylphosphine | | | | | | | | |
| 2) NiTFS - nickel di-trifluoromethylsulphonate; TFSA = trifluoromethylsulphonic acid; PTSA = p-toluenesulphonic acid | | | | | | | | |
| 3) NQ = 1,4-naphthoquinone | | | | | | | | |
| 4) n.d. = not determined | | | | | | | | |

Examples 48-53 are within the scope of Claim 22, and show that good conversions and excellent selectivities are obtained, if a catalyst system in accordance with the invention comprising a promoter as fourth component, is used for the preparation of dimethylsuccinate by carbonylation of methyl acrylate.

In Examples 54 and 55, the catalyst system comprises a monodentate phosphine ligand and no quinone promoter. Low conversion and moderate selectivities were observed. In Example 56, the catalyst system comprises a monodentate phosphine ligand in conjunction with a quinone promoter. However, the addition of a quinone appeared to have a negative effect on the catalyst system of Examples 54 and 55.

Examples 57 and 58 show that for the monocarbonylation of an alkenoic acid derivative in the presence of a nucleophile having a mobile hydrogen atom as coreactant, a quinone promoter is an essential component of a catalyst system containing a bidentate diphosphine ligand.

### Example 59

Using the procedure, equipment and catalyst system of Example 48, a mixture of 20 ml acrylonitrile and 40 ml methanol was reacted at 110 °C for a period of 7 hours. According to the GLC analysis the conversion of acrylonitrile was 5% with a selectivity of 99% into the monomethyl ester mononitrile of malonic acid (methyl cyanoacetate).

## Claims

1. A process for the monocarbonylation of optionally substituted olefinically unsaturated compounds by reaction with carbon monoxide and a coreactant in the presence of a catalyst system comprising:-
a) a source of palladium cations,
b) a bidentate diphosphine, and
c) a source of anions
characterized in that the diphosphine is selected from the group of diphosphines of formula I,
R¹R²P-X-PR³R⁴ (I)
wherein R¹, R², R³ and R⁴ independently represent an optionally substituted aliphatic group, or R¹ together with R² and/or R³ together with R⁴ represent an optionally substituted bivalent aliphatic group, with the proviso of at least one of said monovalent or bivalent aliphatic groups representing an optionally branched or cyclic alkyl or alkylene having at least one alpha hydrogen atom; and X represents a bivalent bridging group containing 1 to 10 atoms in the bridge, with the exception of 1,4-bis(dicyclohexylphosphino)butane.

2. A process as claimed in claim 1, wherein in formula I each of R¹, R², R³ and R⁴ independently represents an unsubstituted optionally branched or cyclic alkyl group having from 1 to 10 carbon atoms.

3. A process as claimed in claims 1 or 2, wherein in formula I at least one of R¹, R², R³ and R⁴ represents an optionally branched alkyl group having one or two alpha hydrogen atoms.

4. A process as claimed in claim 3, wherein in formula I at least one of R¹, R², R³ and R⁴ represents an ethyl, i-propyl, n-propyl, i-butyl, s-butyl or n-butyl group.

5. A process as claimed in any one or more of claims 1-4, wherein the bridging group contains from 2 to 8 atoms in the bridge.

6. A process as claimed in any one or more of claims 1-5, wherein X represents an alkylene chain which is optionally interrupted by one or more oxygen and/or sulphur atoms.

7. A process as claimed in any one or more of claims 1-6, wherein the optionally substituted olefinically unsaturated compound is an alpha-olefin having the formula H₂C=C(A)-Y with A being hydrogen or hydrocarbyl, and Y representing hydrogen or an electron-withdrawing or electron-releasing substituent.

8. A process as claimed in claim 7, wherein Y is selected from the group of carboxy, nitrile, formyl, amino, halo, and substituents of formula -Z-R, wherein Z represents a single valency bond or a functionality -CO-, -COO-, -OOC-, -NH-, -CONH-, -NHCO-, -O-, or -S-; and R represents an optionally substituted alkyl, alkenyl, cycloalkyl, cycloalkenyl, acyl, aryl or heterocyclic group.

9. A process as claimed in any one or more of claims 1-8, wherein a hydride source is used as coreactant.

10. A process as claimed in claim 9, wherein the hydride source is molecular hydrogen.

11. A process as claimed in claim 9 or 10, wherein equimolar amounts of carbon monoxide, hydride source and the optionally substituted olefinically unsaturated compound are brought to reaction to form an aldehyde.

12. A process as claimed in claim 9 or 10, wherein two equivalents of optionally substituted olefinically unsaturated compound per mole of carbon monoxide are brought to reaction to form a ketone.

13. A process as claimed in claim 12, wherein the optionally substituted olefinically unsaturated compound is carbonylated in the presence of a second optionally substituted olefinically unsaturated compound.

14. A process as claimed in any one of claims 9 to 13, wherein the process is carried out under conditions of high hydride activity.

15. A process as claimed in any one or more of claims 1-14, wherein the source of anions is constituted by a strong acid or a salt thereof.

16. A process as claimed in claim 15, wherein the strong acid anions are derived from an acid having a pKa below 3.5.

17. A process as claimed in any one or more of claims 1-8, wherein a nucleophilic compound having a mobile hydrogen atom is used as coreactant.

18. A process as claimed in claim 17, wherein the nucleophilic compound having a mobile hydrogen atom is water, an alcohol, a carboxylic acid, ammonia, an amine, an amide or a thiol.

19. A process as claimed in any one or more of claims 1-7, 17 and 18, wherein the olefinically unsaturated compound is an unsubstituted alpha-olefin.

20. A process as claimed in claim 19, wherein the source of anions is constituted by a weak acid or a salt thereof.

21. A process as claimed in claim 20, wherein the source of anions is constituted by a sterically hindered carboxylic acid.

22. A process as claimed in any one or more claims 1-8, 17 and 18, wherein the olefinically unsaturated compound is an alkenoic acid derivative and the catalyst system further comprises a promoter.

23. A process as claimed in claim 22, wherein the promoter is a quinone promoter selected from the group of benzoquinones, naphthoquinones, anthraquinones and phenanthroquinones.

24. A process as claimed in claim 22 or 23, wherein the anion is a non-coordinating anion derived from a strong acid having a pKa below 2.

25. Use of a catalyst system as defined in claim 1, in the monocarbonylation of optionally substituted olefinically unsaturated compounds.

26. Use of a catalyst system as defined in claim 1 in the preparation of aldehydes by monocarbonylation of an olefinically unsaturated compound in the presence of a hydride source.

27. Use of a catalyst system as defined in claim 1 in the preparation of ketones by monocarbonylation of an olefinically unsaturated compound in the presence of a hydride source.

28. Use of a catalyst system as defined in claim 20 in the preparation of esters by monocarbonylation of an unsubstituted alpha-olefinically unsaturated compound in the presence of an alcohol.

29. Use of a catalyst system as defined in claim 23 in the preparation of alkanedioic acid derivatives by monocarbonylation of an alkenoic acid derivative in the presence of a nuclephilic compound having a mobile hydrogen atom.

30. A catalytic system comprising:-
a) a source of palladium cations,
b) a bidentate diphosphine, and
c) a source of anions
characterized in that the diphosphine is selected from the group of diphosphines of formula I,
R¹R²P-X-PR³R⁴ (I)
wherein R¹, R², R³ and R⁴ independently represent an optionally substituted aliphatic group, or R¹ together with R² and/or R³ together with R⁴ represent an optionally substituted bivalent aliphatic group, with the proviso of at least one of said monovalent or bivalent aliphatic groups representing an optionally branched or cyclic alkyl or alkylene having at least one alpha hydrogen atom; and X represents a bivalent bridging group containing 1 to 10 atoms in the bridge, with the exception of 1,4-bis(dicyclohexylphosphino)butane.

31. A catalyst system as claimed in claim 30, wherein each of R¹, R², R³ and R⁴ represents a secondary alkyl group.

32. A catalyst system as claimed in claim 31, wherein the bidentate diphosphine is 1,3-bis(di-isopropylphosphino)propane.

33. A catalyst system as claimed in claim 30, wherein each of R¹ and R² together and R³ and R⁴ together represents a cycloalkylene group.

34. A catalyst system as claimed in claim 33, wherein the bidentate diphosphine is 1,3-bis(1,5-cyclooctylenephosphino)propane.

## Patentansprüche

1. Verfahren zur Einfachcarbonylierung gegebenenfalls substituierter, olefinisch ungesättigter Verbindungen durch Reaktion mit Kohlenmonoxid und einem weiteren Reaktionspartner in Gegenwart eines Katalysatorsystems aus:
a) einer Palladiumkationenquelle,
b) einem zweizähnigen Diphosphin und
c) einer Anionenquelle,
dadurch gekennzeichnet, daß man das Diphosphin aus der Gruppe der Diphosphine der Formel I,
R¹R²P-X-PR³R⁴ I,
auswählt, wobei R¹, R², R³ und R⁴ voneinander unabhängig für eine gegebenenfalls substituierte aliphatische Gruppe stehen bzw. R¹ zusammen mit R² und/oder R³ zusammen mit R⁴ für eine gegebenenfalls substituierte zweiwertige aliphatische Gruppe stehen, unter der Bedingung, daß mindestens eine der besagten ein- oder zweiwertigen aliphatischen Gruppen für eine gegebenenfalls verzweigte oder cyclische Alkyl- oder Alkylengruppe mit mindestens einem alpha-Wasserstoffatom steht, und X für eine zweiwertige Brückengruppe mit 1 bis 10 Kohlenstoffatomen in der Brücke steht, mit Ausnahme von 1,4-Bis(dicyclohexylphosphino)butan.

2. Verfahren nach Anspruch 1, bei dem in Formel I R¹, R², R³ und R⁴ jeweils voneinander unabhängig für eine unsubstituierte, gegebenenfalls verzweigte oder cyclische Alkylgruppe mit 1 bis 10 Kohlenstoffatomen stehen.

3. Verfahren nach Anspruch 1 oder 2, bei dem in Formel I mindestens einer der Reste R¹, R², R³ und R⁴ für eine gegebenenfalls verzweigte Alkylgruppe mit einem oder zwei alpha-Wasserstoffatomen steht.

4. Verfahren nach Anspruch 3, bei dem in Formel I mindestens einer der Reste R¹, R², R³ und R⁴ für eine Ethyl-, i-Propyl, n-Propyl-, i-Butyl-, s-Butyl- oder n-Butylgruppe steht.

5. Verfahren nach einem der Ansprüche 1-4, bei dem die Brückengruppe 2 bis 8 Atome in der Brücke enthält.

6. Verfahren nach einem der Ansprüche 1-5, bei dem X für eine Alkylenkette steht, die gegebenenfalls durch ein oder mehrere Sauerstoff- und/oder Schwefelatome unterbrochen ist.

7. Verfahren nach einem der Ansprüche 1-6, bei dem die gegebenenfalls substituierte, olefinisch ungesättigte Verbindung ein alpha-Olefin mit der Formel H₂C=C(A)-Y, wobei A Wasserstoff oder ein Kohlenwasserstoffrest ist und Y für Wasserstoff oder einen elektronenanziehenden oder elektronenabstoßenden Substituenten steht, darstellt.

8. Verfahren nach Anspruch 7, bei dem man Y aus der Gruppe Carboxy, Nitril, Formyl, Amino, Halogen und Substituenten der Formel -Z-R, wobei Z für eine Einfachbindung oder eine Funktionalität -CO-, -COO-, -OOC-, -NH-, -CONH-, -NHCO-, -O- oder -S- und R für eine gegebenenfalls substituierte Alkyl-, Alkenyl-, Cycloalkyl-, Cycloalkenyl-, Acyl-, Aryl- oder heterocyclische Gruppe steht, auswählt.

9. Verfahren nach einem der Ansprüche 1-8, bei dem eine Hydridquelle als weiterer Reaktionspartner verwendet wird.

10. Verfahren nach Anspruch 9, bei dem die Hydridquelle molekularer Wasserstoff ist.

11. Verfahren nach Anspruch 9 oder 10, bei dem man äquimolare Mengen an Kohlenmonoxid, Hydridquelle und der gegebenenfalls substituierten, olefinisch ungesättigten Verbindung zur Bildung eines Aldehyds zur Reaktion bringt.

12. Verfahren nach Anspruch 9 oder 10, bei dem man zwei Äquivalente der gegebenenfalls substituierten, olefinisch ungesättigten Verbindung pro Mol Kohlenmonoxid zur Bildung eines Ketons zur Reaktion bringt.

13. Verfahren nach Anspruch 12, bei dem die gegebenenfalls substituierte, olefinisch ungesättigte Verbindung in Gegenwart einer zweiten gegebenenfalls substituierten, olefinisch ungesättigten Verbindung carbonyliert wird.

14. Verfahren nach einem der Ansprüche 9 bis 13, bei dem man das Verfahren unter Bedingungen hoher Hydridaktivität durchführt.

15. Verfahren nach einem der Ansprüche 1-14, bei dem die Anionenquelle aus einer starken Säure oder einem Salz derselben besteht.

16. Verfahren nach Anspruch 15, bei dem die Anionen einer starken Säure sich von einer Säure mit einem pKa-Wert kleiner 3,5 ableiten.

17. Verfahren nach einem der Ansprüche 1-8, bei dem man eine nukleophile Verbindung mit einem beweglichen Wasserstoffatom als weiteren Reaktionspartner verwendet.

18. Verfahren nach Anspruch 17, bei dem die nukleophile Verbindung mit einem beweglichen Wasserstoffatom Wasser, ein Alkohol, eine Carbonsäure, Ammoniak, ein Amin, ein Amid oder ein Thiol ist.

19. Verfahren nach einem der Ansprüche 1-7, 17 und 18, bei dem die olefinisch ungesättigte Verbindung ein unsubstituiertes alpha-Olefin ist.

20. Verfahren nach Anspruch 19, bei dem die Anionenquelle aus einer schwachen Säure oder einem Salz derselben besteht.

21. Verfahren nach Anspruch 20, bei dem die Anionenquelle aus einer sterisch gehinderten Carbonsäure besteht.

22. Verfahren nach einem der Ansprüche 1-8, 17 und 18, bei dem die olefinisch ungesättigte Verbindung ein Alkensäurederivat ist und das Katalysatorsystem zusätzlich einen Promotor enthält.

23. Verfahren nach Anspruch 22, bei dem der Promotor ein Chinonpromotor ist, ausgewählt aus der Gruppe der Benzochinone, Naphthochinone, Anthrachinone und Phenanthrochinone.

24. Verfahren nach Anspruch 22 oder 23, bei dem das Anion ein von einer starken Säure mit einem pKa-Wert kleiner 2 abgeleitetes, nichtkoordinierendes Anion ist.

25. Verwendung eines Katalysatorsystems nach Anspruch 1 bei der Einfachcarbonylierung von gegebenenfalls substituierten, olefinisch ungesättigten Verbindungen.

26. Verwendung eines wie in Anspruch 1 definierten Katalysatorsystems bei der Herstellung von Aldehyden durch Einfachcarbonylierung einer olefinisch ungesättigten Verbindung in Gegenwart einer Hydridquelle.

27. Verwendung eines wie in Anspruch 1 definierten Katalysatorsystems bei der Herstellung von Ketonen durch Einfachcarbonylierung einer olefinisch ungesättigten Verbindung in Gegenwart einer Hydridquelle.

28. Verwendung eines wie in Anspruch 20 definierten Katalysatorsystems bei der Herstellung von Estern durch Einfachcarbonylierung einer unsubstituierten, alpha-olefinisch ungesättigten Verbindung in Gegenwart eines Alkohols.

29. Verwendung eines wie in Anspruch 23 definierten Katalysatorsystems bei der Herstellung von Alkandisäurederivaten durch Einfachcarbonylierung eines Alkensäurederivats in Gegenwart einer nukleophilen Verbindung mit einem beweglichen Wasserstoffatom.

30. Ein Katalysatorsystem aus:-
a) einer Palladiumkationenquelle,
b) einem zweizähnigen Diphosphin und
c) einer Anionenquelle,
dadurch gekennzeichnet, daß man das Diphosphin aus der Gruppe der Diphosphine der Formel I,
R¹R²P-X-PR³R⁴ (I)
auswählt, wobei R¹, R², R³ und R⁴ voneinander unabhängig für eine gegebenenfalls substituierte aliphatische Gruppe stehen bzw. R¹ zusammen mit R² und/oder R³ zusammen mit R⁴ für eine gegebenenfalls substituierte zweiwertige aliphatische Gruppe stehen, unter der Bedingung, daß mindestens eine der besagten ein- oder zweiwertigen aliphatischen Gruppen für eine gegebenenfalls verzweigte oder cyclische Alkyl- oder Alkylengruppe mit mindestens einem alpha-Wasserstoffatom steht, und X für eine zweiwertige Brückengruppe mit 1 bis 10 Kohlenstoffatomen in der Brücke steht, mit Ausnahme von 1,4-bis(Dicyclohexylphosphino)butan.

31. Katalysatorsystem nach Anspruch 30, bei dem R¹, R², R³ und R⁴ jeweils für eine sekundäre Alkylgruppe stehen.

32. Katalysatorsystem nach Anspruch 31, bei dem das zweizähnige Diphosphin 1,3-bis(Di-Isopropylphosphino)propan ist.

33. Katalysatorsystem nach Anspruch 30, bei dem jeweils R¹ und R² zusammen sowie R³ und R⁴ zusammen für eine Cycloalkylengruppe stehen.

34. Katalysatorsystem nach Anspruch 33, bei dem das zweizähnige Diphosphin 1,3-bis(1,5-Cyclooctylenphosphino)propan ist.

## Revendications

1. Procédé de monocarbonylation de composés oléfiniquement insaturés, facultativement substitués, par réaction avec du monoxyde de carbone et un coréactif en présence d'un système catalytique comprenant :
a) une source de cations palladium;
b) une diphosphine bidentate, et
c) une source d'anion,
caractérisé en ce que la diphosphine est choisie parmi le groupe de diphosphines de formule (I) :
R¹R²P-X-PR³R⁴ (I)
dans laquelle R¹, R², R³ et R⁴ représentent, indépendamment, un radical aliphatique facultativement substitué, ou R¹ avec R² et/ou R³ avec R⁴ représentent ensemble un radical aliphatique bivalent facultativement substitué, à la condition qu'au moins l'un des radicaux aliphatiques monovalents ou bivalents représente un alkyle ou alkylène facultativement ramifié ou cyclique, ayant au moins un atome d'hydrogène en alpha, et X représente un groupe pontant bivalent contenant de 1 à 10 atomes dans le pont, à l'exception du 1,4-bis(dicyclohexylphosphino)butane.

2. Procédé suivant la revendication 1, dans lequel, dans la formule (I), chacun des R¹, R², R³ et R⁴ représente indépendamment, un radical alkyle non substitué, facultativement ramifié ou cyclique, ayant de 1 à 10 atomes de carbone.

3. Procédé suivant la revendication 1 ou 2, dans lequel, dans la formule (I), au moins l'un des R¹, R², R³ et R⁴ représente un radical alkyle facultativement ramifié ayant un ou deux atomes d'hydrogène en alpha.

4. Procédé suivant la revendication 3, dans lequel, dans la formule (I), au moins l'un des R¹, R², R³ et R⁴ représente un radical éthyle, i-propyle, n-propyle, i-butyle, s-butyle ou n-butyle.

5. Procédé suivant l'une quelconque ou plusieurs des revendications 1 à 4, dans lequel le groupe pontant contient de 2 à 8 atomes dans le pont.

6. Procédé suivant l'une quelconque ou plusieurs des revendications 1 à 5, dans lequel X représente une chaîne alkylène qui est facultativement interrompue par un ou plusieurs atomes d'oxygène et/ou de soufre.

7. Procédé suivant l'une quelconque ou plusieurs des revendications 1 à 6, dans lequel le composé oléfiniquement insaturé, facultativement substitué est une alpha-oléfine ayant le formule H₂C=C(A)-Y avec A étant hydrogène ou hydrocarbyle et Y représentant hydrogène ou un substituant électrocapteur ou électrodonneur.

8. Procédé suivant la revendication 7, dans lequel Y est choisi parmi le groupe des carboxy, nitrile, formyle, amino, halo et les substituants de formule -Z-R, où Z représente une liaison à valence simple ou une fonctionnalité -CO-, -COO-, -OOC-, -NH-, -CONH-, -NHCO-, -O-ou -S- et R représente un radical alkyle, alcényle, cycloalkyle, cycloalcényle, acyle, aryle ou hétérocyclique facultativement substitué.

9. Procédé suivant l'une quelconque ou plusieurs des revendications 1 à 8, dans lequel une source d'hydrure est utilisées comme coréactif.

10. Procédé suivant la revendication 9, dans lequel la source d'hydrure est de l'hydrogène moléculaire.

11. Procédé suivant la revendication 9 ou 10, dans lequel des quantités équimolaires de monoxyde de carbone, de source d'hydrure et du composé oléfiniquement insaturé, facultativement substitué sont mises à réagir pour former un aldéhyde.

12. Procédé suivant la revendication 9 ou 10, dans lequel deux équivalents du composé oléfiniquement insaturé, facultativement substitué par mole de monoxyde de carbone sont mis à réagir pour former une cétone.

13. Procédé suivant la revendication 12, dans lequel le composé oléfiniquement insaturé, facultativement substitué est carbonylé en présence d'un second composé oléfiniquement insaturé, facultativement substitué.

14. Procédé suivant l'une quelconque des revendications 9 à 13, où le procédé est réalisé dans des conditions d'activité élevée des hydrures.

15. Procédé suivant l'une quelconque ou plusieurs des revendications 1 à 14, dans lequel la source d'anion est constituée d'un acide fort ou d'un sel de celui-ci.

16. Procédé suivant la revendication 15, dans lequel les anions de l'acide fort proviennent d'un acide ayant un pKa inférieur à 3,5.

17. Procédé suivant l'une quelconque ou plusieurs des revendications 1 à 8, dans lequel un composé nucléophile ayant un hydrogène mobile est utilisé comme coréactif.

18. Procédé suivant la revendication 17, dans lequel le composé nucléophile ayant un hydrogène mobile est l'eau, un alcool, un acide carboxylique, l'ammoniac, une amine, un amide ou un thiol.

19. Procédé suivant l'une quelconque ou plusieurs des revendications 1 à 7, 17 et 18, dans lequel le composé oléfiniquement insaturé, facultativement substitué est une alpha-oléfine non substituée.

20. Procédé suivant la revendication 19, dans lequel la source d'anion est constituée par un acide faible ou un sel de celui-ci.

21. Procédé suivant la revendication 20, dans lequel la source d'anion est constituée par un acide carboxylique stériquement encombré.

22. Procédé suivant l'une quelconque ou plusieurs des revendications 1 à 8, 17 et 18, dans lequel le composé oléfiniquement insaturé est un dérivé d'acide alcénoïque et le système catalytique comprend, en outre, un promoteur.

23. Procédé suivant la revendication 22, dans lequel le promoteur est un promoteur quinonique choisi parmi le groupe des benzoquinones, naphtoquinones, anthraquinones et phénanthroquinones.

24. Procédé suivant la revendication 22 ou 23, dans lequel l'anion est un anion non coordinant provenant d'un acide fort ayant un pKa inférieur à 2.

25. Utilisation d'un système catalytique tel que défini à la revendication 1, dans la monocarbonylation de composés oléfiniquement insaturés, facultativement substitués.

26. Utilisation d'un système catalytique tel que défini à la revendication 1, dans la préparation d'aldéhydes par monocarbonylation d'un composé oléfiniquement insaturé en présence d'une source d'hydrure.

27. Utilisation d'un système catalytique tel que défini à la revendication 1, dans la préparation de cétones par monocarbonylation d'un composé oléfiniquement insaturé, en présence d'une source d'hydrure.

28. Utilisation d'un système catalytique tel que défini à la revendication 20, dans la préparation d'esters par monocarbonylation d'un composé alpha-oléfiniquement insaturé non substitué, en présence d'un alcool.

29. Utilisation d'un système catalytique tel que défini à la revendication 23, dans la préparation de dérivés d'acide alcanedioïque par monocarbonylation de dérivés d'acide alcénoïque, en présence d'un composé nucléophile ayant un atome d'hydrogène mobile.

30. Système catalytique comprenant :
a) une source de cations palladium;
b) une diphosphine bidentate, et
c) une source d'anion,
caractérisé en ce que la diphosphine est choisie parmi le groupe de diphosphines de formule (I) :
R¹R²P-X-PR³R⁴ (I)
dans laquelle R¹, R², R³ et R⁴ représentent, indépendamment, un radical aliphatique facultativement substitué, ou R¹ avec R² et/ou R³ avec R⁴ représentent ensemble un radical aliphatique bivalent facultativement substitué, à la condition qu'au moins l'un des radicaux aliphatiques monovalents ou bivalents représente un alkyle ou alkylène facultativement ramifié ou cyclique, ayant au moins un atome d'hydrogène en alpha, et X représente un groupe pontant bivalent contenant de 1 à 10 atomes dans le pont, à l'exception du 1,4-bis(dicyclohexylphosphino)butane.

31. Système catalytique suivant la revendication 30, dans lequel chacun des R¹, R², R³ et R⁴ représente un radical alkyle secondaire.

32. Système catalytique suivant la revendication 31, dans lequel la diphosphine bidentate est le 1,3-bis(di-isopropylphosphino)propane.

33. Système catalytique suivant la revendication 30, dans lequel chacun des R¹ et R² ensemble et R³ et R⁴ ensemble représente un radical cycloalkylène.

34. Système catalytique suivant la revendication 33, dans lequel la diphosphine bidentate est le p1,3-bis(1,5-cyclooctylènephosphino)propane.
